# EUROPEAN PATENT APPLICATION

(11) **EP 4 803 881 A1**
(43) Date of publication of application: **09.09.2026**
(21) Application number: 24885589.2
(22) Date of filing: 24.10.2024
(51) Int. Cl.: G01N 17/00, C23C 2/00

(54) **PREDICTION DEVICE, PREDICTION METHOD, AND PREDICTION PROGRAM**

(30) Priority: 31.10.2023 JP 2023186777
(71) Applicant: Nippon Steel Corporation, Tokyo 100-8071 (JP)
(72) Inventor: TOKUDA Kohei, Tokyo 100-8071 (JP); GOTO Yasuto, Tokyo 100-8071 (JP); KAWAMOTO Kosuke, Tokyo 100-8071 (JP)
(74) Representative: Zimmermann & Partner Patentanwälte mbB
(86) International application number: PCT/JP2024/037961
(87) International publication number: WO 2025/094816

(57) **Abstract**

This prediction device includes a prediction unit in which the prediction unit is configured to calculate, based on respective contents of multiple components contained in a coating layer, a first proportion during a first period and a second proportion during a second period, for each phase constituting the coating layer, calculate, based on a corrosion order of the each phase, the first proportion of the each phase, and a first corrosion rate of the each phase, a duration of the first period until a part of corrosion of the coating layer reaches a base steel, calculate, based on a corrosion order of the each phase, the second proportion of the each phase, and a second corrosion rate of the each phase, a duration of the second period following the first period, and calculate a sum of the duration of the first period and the duration of the second period as a prediction result of corrosion resistance of the coating layer.

## Description

### TECHNICAL FIELD

The present invention relates to a prediction device, a prediction method, and a prediction program.

Priority is claimed on Japanese Patent Application No. 2023-186777 filed on October 31, 2023, the content of which is incorporated herein by reference.

### BACKGROUND ART

Since steel corrodes when exposed to the atmosphere, corrosion-resistant treatments such as coating are necessary for steel to be used over a long period. Coated steel is less expensive than stainless steel. In recent years, from the perspective of carbon neutrality, life cycle cost has become increasingly important, and high corrosion-resistant coated steel sheets capable of protecting steel over the long term have attracted attention. Examples of high corrosion-resistant coated steel sheets include zinc-aluminum-magnesium (Zn-Al-Mg) alloy coated steel sheets (see Patent Documents 1 and 2).

### Citation List

### Patent Documents

Patent Document 1: Japanese Patent Publication No. 7136351
Patent Document 2: Japanese Patent Publication No. 7040695

### SUMMARY OF INVENTION

### Technical Problem

Various properties such as corrosion resistance, sacrificial corrosion resistance, workability, wear resistance, and color tone are required for coated steel sheets. However, the properties of coated steel sheets are evaluated independently by each steel company and are not uniformly assessed across all steel companies. Consequently, end users may find it difficult to select the optimal coated steel sheet based on these properties.

Furthermore, these properties depend significantly on the alloy composition of the coating layer. That is, the properties of the coating layer depend on the material (phase) formed by the bonding of the elements of each metal component and on the content of that material. For example, a Zn-Al coating layer contains both a Zn phase and an Al phase. Here, increasing the Al content leads to a higher proportion of the Al phase within the coating layer. Consequently, the properties of the coating layer approach those of Al, improving its corrosion resistance. On the other hand, the sacrificial corrosion resistance, which is excellent in the Zn phase, becomes less effective in coating layers with a reduced Zn phase content.

Furthermore, knowing the corrosion resistance of coated steel sheets enables end users to select the optimal coated steel sheet with a focus on lifecycle costs. Additionally, knowing the corrosion resistance of coated steel sheets reduces uncertainty regarding maintenance and replacement frequency of the coated steel sheets. Consequently, many users are interested in the corrosion resistance (service life, lifespan) of coated steel sheets.

To improve the accuracy of predicting the corrosion resistance of coating layers, it is desirable to calculate the corrosion rate based on pre-obtained exposure test results and accelerated corrosion test results for various coated steel sheets. However, in recent years, coating layers may contain not only Al and Zn but also Mg. Since the corrosion resistance of Al, Zn, and Mg differs, predicting the corrosion resistance of the coating layer has become more difficult. Thus, there is a problem in that the accuracy of predicting the corrosion resistance of the coating layer cannot be improved.

In light of the above circumstances, the present invention aims to provide a prediction device, a prediction method, and a prediction program capable of improving the accuracy of predicting the corrosion resistance of coating layers.

### Solution to Problem

One aspect of the present invention is a prediction device comprising a prediction unit, in which the prediction unit is configured to: calculate, based on respective contents of multiple components contained in a coating layer, a first proportion during a first period and a second proportion during a second period, for each phase constituting the coating layer, calculate, based on a corrosion order of the each phase, the first proportion of the each phase, and a first corrosion rate of the each phase, a duration of the first period until a part of corrosion of the coating layer reaches a base steel, calculate, based on a corrosion order of the each phase, the second proportion of the each phase, and a second corrosion rate of the each phase, a duration of the second period following the first period, and calculate a sum of the duration of the first period and the duration of the second period as a prediction result of corrosion resistance of the coating layer.

One aspect of the present invention is a prediction method executed by a prediction device, the method including steps of calculating, based on respective contents of multiple components contained in a coating layer, a first proportion during a first period and a second proportion during a second period, for each phase constituting the coating layer, calculating, based on a corrosion order of the each phase, the first proportion of the each phase, and a first corrosion rate of the each phase, a duration of the first period until a part of corrosion of the coating layer reaches a base steel, calculating, based on a corrosion order of the each phase, the second proportion of the each phase, and a second corrosion rate of the each phase, a duration of the second period following the first period, and calculating a sum of the duration of the first period and the duration of the second period as a prediction result of corrosion resistance of the coating layer.

One aspect of the present invention is a prediction program for causing a computer to execute processes of calculating, based on respective contents of multiple components contained in a coating layer, a first proportion during a first period and a second proportion during a second period, for each phase constituting the coating layer, calculating, based on a corrosion order of the each phase, the first proportion of the each phase, and a first corrosion rate of the each phase, a duration of the first period until a part of corrosion of the coating layer reaches a base steel, calculating, based on a corrosion order of the each phase, the second proportion of the each phase, and a second corrosion rate of the each phase, a duration of the second period following the first period, and calculating a sum of the duration of the first period and the duration of the second period as a prediction result of corrosion resistance of the coating layer. Advantageous Effects of Invention

According to the present invention, it is possible to improve the accuracy in predicting the corrosion resistance of a coating layer.

### BRIEF DESCRIPTION OF DRAWINGS

[FIG. 1] A diagram showing a configuration example of a prediction device according to an embodiment.
[FIG. 2] A view showing an example of a model of the corrosion morphology of the Zn-Al-Mg coating layer in the embodiment.
[FIG. 3] A diagram showing an example of liquidus temperature of Zn-Al-Mg in the embodiment.
[FIG. 4] A diagram showing an example of the one mole molecular weight and density of each phase in the embodiment.
[FIG. 5] A diagram showing an example of a database of the area fraction of each phase in the embodiment.
[FIG. 6] A flowchart showing an example of the process for calculating the coating weight of each phase in the embodiment.
[FIG. 7] A diagram showing an example of the corrosion rate for each phase during the first period in the embodiment.
[FIG. 8] A flowchart showing an example of a process for calculating the duration of the period (first period) during which partial corrosion can be observed on the surface of the coating layer, and the duration of the period (second period) after partial corrosion reaches the interface between the coating layer and the steel sheet in the embodiment.

### DESCRIPTION OF EMBODIMENTS

The embodiments of the present invention will now be described in detail with reference to the accompanying drawings.

Hereinbelow, a prediction device, a prediction method, and a prediction program will be described using a coating layer of zinc "Zn"-aluminum "Al"-magnesium "Mg" as an example of a coating layer; however, the coating layer in the embodiment is not limited to the Zn-Al-Mg coating layer.

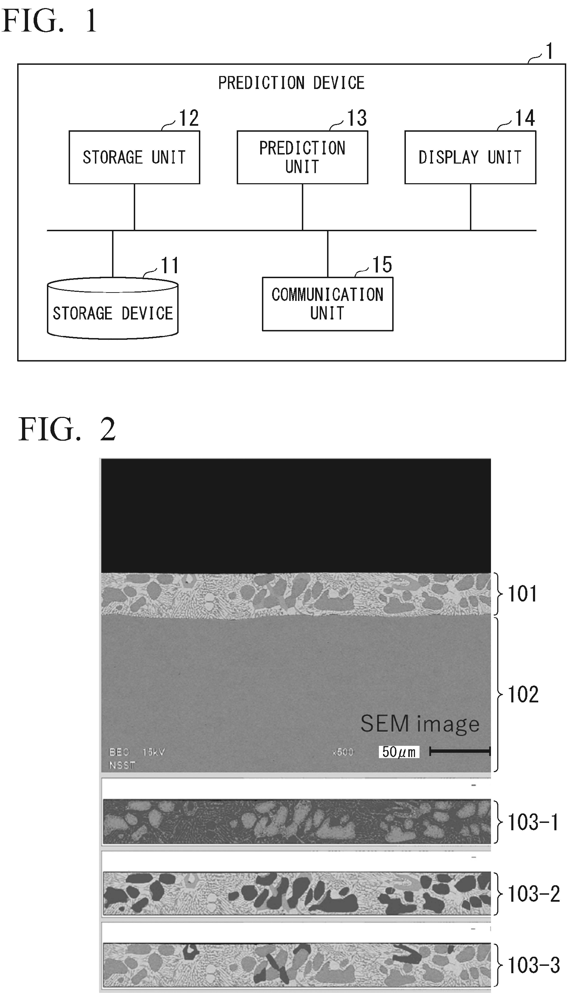

FIG. 1 is a diagram showing an example configuration of a prediction device 1 according to an embodiment. The prediction device 1 is an information processing device that predicts the corrosion resistance of a coating layer, such as a personal computer or a smartphone terminal. The prediction device 1 includes a storage device 11, a storage unit 12, a prediction unit 13, a display unit 14, and a communication unit 15.

The storage device 11 is a non-volatile recording medium (non-transitory recording medium). The prediction program is loaded from the storage device 11 into the storage unit 12.

The prediction unit 13 is, for example, a processor such as a CPU (central processing unit). The prediction unit 13 is implemented as software by executing a prediction program loaded from the storage device 11 into the storage unit 12. The prediction program may be recorded on a computer-readable recording medium. The computer-readable recording medium is, for example, a non-transitory recording medium including a portable medium such as a flexible disk, a magneto-optical disk, a ROM (read-only memory), and a CD-ROM (compact disc read-only memory), and a storage device such as a hard disk and an SSD (solid-state drive) built into computer systems.

The prediction unit 13 may be implemented using hardware, such as electronic circuits or circuitry employing an LSI (large-scale integration circuit), an ASIC (application-specific integrated circuit), a PLD (programmable logic device), or an FPGA (field-programmable gate array).

The prediction unit 13 predicts the corrosion resistance of the coating layer based on the respective contents of Zn, Al, and Mg (each input value). For example, the prediction unit 13 calculates the proportion of each phase constituting the coating layer and predicts the life time (service life) of the coating layer based on the calculated proportions.

The display unit 14 includes a display device such as a liquid-crystal display. The display unit 14 displays, for example, prediction results.

The communication unit 15 performs communication with an external communication device (not shown) via communication lines such as the Internet, a LAN (local area network), or a WAN (wide area network). The communication unit 15 may, for example, transmit prediction results to that communication device (not shown).

Next, details of the prediction device, the prediction method, and the prediction program will be described.

Ideally, the service life of coated steel sheets should be predicted based on a database of exposure test results of the coated steel sheets. However, coated steel sheets used in the building materials sector require a service life (corrosion resistance) of 30 to 50 years or more. Consequently, creating a database for recently developed coated steel sheets is difficult in practice. Nevertheless, it is possible to predict the corrosion resistance of coated steel sheets in exposure tests by accurately predicting the corrosion resistance of the coating layer based on the results (findings) of corrosion acceleration tests (e.g., composite cycle corrosion tests) that show a certain degree of correlation with exposure tests.

Therefore, the prediction device 1 predicts the corrosion resistance of the coating layer based on accumulated knowledge regarding corrosion acceleration, which has a certain degree of correlation with exposure tests. By changing the salt concentration and its cycle interval, it is possible to alter the acceleration rate (corrosion rate) of the corrosion acceleration test. Furthermore, if the corrosion rate of each material (phase) constituting the coating layer can be obtained, it becomes possible to predict the corrosion resistance of the coated steel sheet.

Furthermore, the prediction device 1 predicts the corrosion resistance of the coating layer using a corrosion model based on findings obtained from numerous corrosion acceleration tests. For the corrosion resistance (service life) of the coating layer to be calculated, the corrosion morphology of the coating layer must be sufficiently understood theoretically, and the findings on the corrosion morphology must be reflected in the corrosion model. In this regard, the relationship between the corrosion model and corrosion acceleration tests, as well as the relationship between the corrosion model and exposure tests, has been sufficiently confirmed.

FIG. 2 is view (a cross-sectional view) showing an example of a model (corrosion model) of a corrosion morphology for the Zn-Al-Mg coating layer in the embodiment. A coating layer 101 is a Zn-Al-Mg coating layer. The coating layer 101 provides sacrificial corrosion resistance to a steel 102 by being present on its surface. For example, the region of the ternary eutectic structure is shown in a coating layer 103-1.

During the corrosion period including Stage I (first period) and Stage II (second period) as described below, the coating layer 101 (Zn-Al-Mg coating layer) corrodes depending on the usage environment.

Stage I: A period during which the coating layer undergoes partial corrosion starting from the surface of the coating layer.

Stage II: A period during which a part of corrosion of the coating layer reaches the interface between the coating layer and the steel, and the coating layer continues to corrode while providing sacrificial corrosion resistance to the steel (base steel). The steel sheet remains protected until the coating layer, which provides sacrificial corrosion resistance to the base steel sheet, is consumed.

The end of Stage II corresponds to the end of the service life of the coating layer. When Stage II ends, localized red rust formed on the steel by corrosion becomes visible on the surface of the coating layer. The prediction device 1 calculates the number of cycles required for the coating weight or thickness of the coating layer provided as an initial input to reach 0 due to the decreasing of the coating weight of the coating layer remaining on the steel sheet surface during the corrosion acceleration test, and uses this number of cycles as the service life (corrosion resistance) of the coating layer.

The reason for separating the corrosion period (service life) into Stage I and Stage II is that, in Stage I, the corrosion rate is inherent to each phase constituting the coating layer. In contrast, in Stage II, corrosion of the coating layer is accelerated due to protection of the steel sheet (base steel). Therefore, in Stage II, a corrected corrosion rate (second corrosion rate) for each phase is used, which is obtained by multiplying the corrosion rate in Stage I (first corrosion rate) by an acceleration factor.

The prediction device 1 calculates the corrosion rate of each phase constituting the coating layer for each period of Stage I and Stage II, based on the composition of the coating layer (input value). The prediction device 1 calculates the overall corrosion resistance (service life) of the coating layer by summing each service life corresponding to the corrosion rate of each phase.

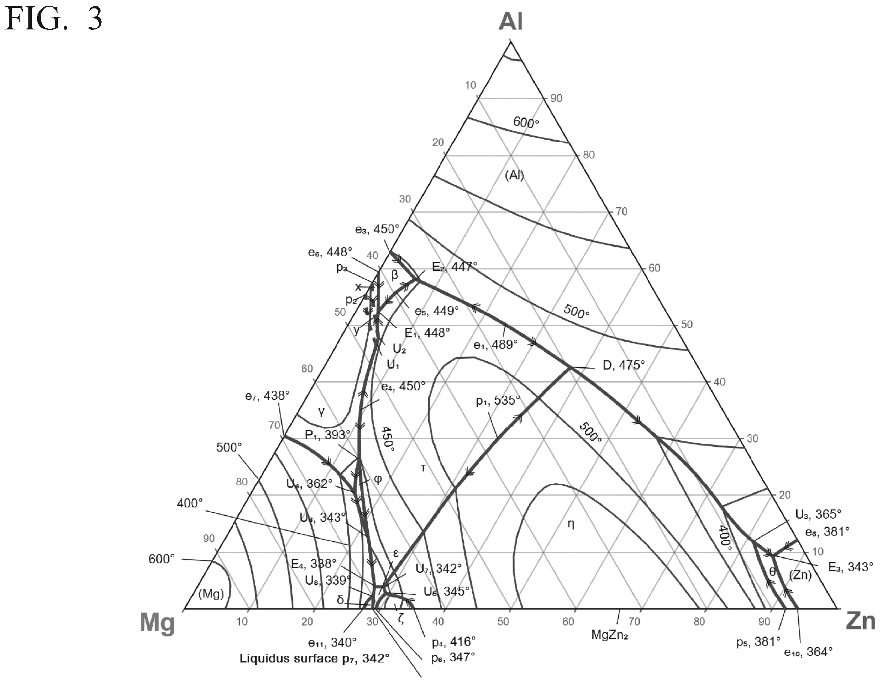

FIG. 3 shows a diagram (liquidus diagram) illustrating an example of the liquidus temperature of Zn-Al-Mg (Reference Document 1: P. Liang, T. Tarfa, J.A. Robinson, et al., "Experimental investigation and thermodynamic calculation of the Al±Mg±Zn system," Thermochimica Acta 314 (1998) 87-110) in the embodiment.

The liquidus lines in the liquidus diagram shown in FIG. 3 correspond to contour lines on a map. For example, the thick liquidus line in the liquidus diagram shown in FIG. 3 corresponds to a valley line on a map. The thick liquidus line represents the solidification process of the Zn-Al-Mg alloy. Specifically, in the liquidus diagram illustrated in FIG. 3, when predetermined positions are input into the prediction device 1 as initial values for the respective Zn-Al-Mg content levels, the solidification of the Zn-Al-Mg alloy proceeds along the thick liquidus line near that position, with the temperature decreasing.

### (Input)

Information regarding the coating layer for which corrosion resistance (service life) is to be calculated is provided to the prediction unit 13. Specifically, to confirm changes in corrosion resistance due to differences in the composition of the Zn-Al-Mg coating layer, the Al and Mg components are provided to the prediction unit 13 in either "at%" or "mass%". The total sum of the Zn, Al, and Mg component proportion is 100%. Therefore, it can be expressed as "(Zn at%, Al at%, Mg at%) = (100 - x - y, x, y)". The atomic weights of Zn, Al, and Mg are Zn = 65.38, Al = 26.98, Mg = 24.305. Using these, the "mass%" equivalent to "at%" is calculated.

Furthermore, the densities of the Zn, Al, and Mg components are provided to the prediction unit 13. The prediction unit 13 calculates the theoretical density "ρ" based on the densities of the Zn, Al, and Mg components. Here, the density of Zn is "ρZn = 7.14 g/cm³", the density of Al is "ρAl = 2.7 g/cm³", and the density of Mg is "ρMg = 1.738 g/cm³". The prediction unit 13 calculates the theoretical density "ρ" based on the density of each component and the "mass%".

In addition to the composition of the coating layer to be predicted, the coating layer's thickness "Lµm" and the coating layer's coating weight must be provided to the prediction unit 13. The coating weight of the coating layer is, for example, approximately 5 to 100 µm and 20 to 630 g/m². When converting between coating thickness and coating weight, multiplying the coating thickness by the theoretical density "ρ" yields the coating weight. Conversely, dividing the coating weight by the specific gravity yields the coating thickness [µm].

In the prediction calculation of the embodiment, numerical values concerning the coating weight are used. Here, "coating weight = ρL g/m²" is provided to the prediction unit 13. For example, for 100% Zn coating, the coating thickness equivalent to 100 g/m² is "100 ÷ 7.12 ≈ 14 µm".

The thinner the coating layer, the shorter its service life. If the coating layer is thick, even if a significant portion corrodes, its sufficient thickness allows Stage I "τ1" to last longer, delaying the start of Stage II "τ2". Conversely, if the coating layer is thin, Stage I "τ1" becomes shorter, causing Stage II "τ2" to start earlier.

In Zn-Al-Mg coating layers with a thickness of 20 µm, Stage II "τ2" tends to start when approximately 30% of the coating layer has corroded. In Zn-Al-Mg coating layers with a thickness of 30 µm, Stage II "τ2" tends to start when 50% of the entire coating layer has corroded. Therefore, considering the proportion of the coating layer at the timing of starting Stage II "τ2" as (= 1 - G), the proportion of the coating layer "G = 0.0 to 1.0" corroded in Stage I "τ1" should be input into the prediction unit 13. Assuming that the coating layer structure is homogeneous, Equations (1) and (2) are established based on the proportion "G" and the coating weight "pL".$TM 1 = ρL G$ $TM 2 = ρL \left(1-G\right)$

Here, the "TM1" represents the coating weight (corrosion weight loss) of the coating layer corroded during Stage I "τ1". The "TM2" represents the coating weight (corrosion weight loss) of the coating layer corroded during Stage II "τ2".

The corrosion resistance of a coating layer varies depending on the proportion of each phase (constituent phase) within the coating layer. An increase in the proportion of highly corrosion-resistant phases (phases with slower corrosion rates) extends the service life of the coating layer. Note that the corrosion resistance referred to here is the corrosion weight loss (decreased amount) of the pure coating layer itself, excluding the steel. This corrosion resistance is distinct from the effect of the coating layer corroding instead of the steel to protect the steel (sacrificial corrosion resistance).

If the coating layer contains Zn, Al, and Mg components, the phases constituting the coating layer can be classified into six types. Note that this classification into six types (the 1st phase to the 6th phase) follows the solidification process of Zn, Al, and Mg alloys. Therefore, the ranges (input ranges) for the respective Zn, Al, and Mg contents are restricted. For example, the input range for the Zn component is "25 to 85 at%", the input range for the Al component is "10 to 73 at%", and the input range for the Mg component is "2 to 18%".

Examples of the definitions for each phase are shown below.

The 1st phase, "Pure_Al phase", is a phase containing trace amount of Zn (content of "16.5 at% or less") and Al.

The 2nd phase, "Al-Zn(α) phase", is a mixed phase of the Al phase and the Zn phase (Zn content is 16.5 at%).

The 3rd phase, "Zn-Al(β) phase", is a mixed phase of the Zn phase and the Al phase (Zn content is 59 at%).

The 4th phase, "Pure_Zn(η) phase", is a phase containing trace amount of Al (content of "16.5 at% or less") and Zn (Zn content is 98.4 to 100 at%).

The 5th phase, "MgZn2 phase", is a single phase (crystallite) formed either simultaneously with or prior to the formation of the Pure_Al phase from the liquid phase. Typically, the size of the "MgZn2 phase" is 1 µm or larger.

The 6th phase, "ternary eutectic structure", is a phase composed of fine Pure_Al, fine Pure_Zn, and fine MgZn2, forming a mixed structure via ternary eutectic reaction. The overall composition is "Zn-9at%Al-5at%Mg".

By these definitions based on the atomic composition of each phase, it is possible to perform theoretical calculations of the molecular weight and density for each phase.

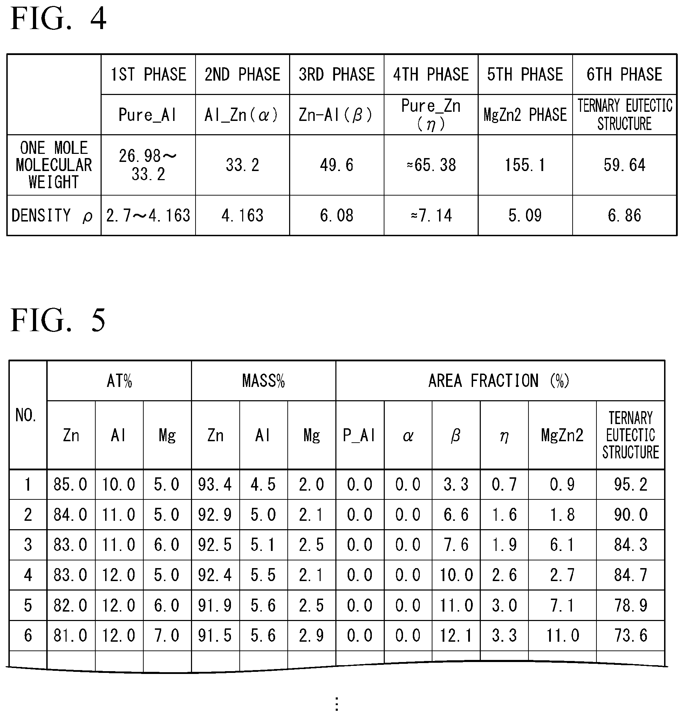

FIG. 4 shows examples of one mole molecular weight and density of each phase in the embodiment. When the component composition of the coating layer and the molecular weight of the constituent phase of each phase are given, it is possible to calculate the weight and molar quantity of each phase constituting the coating layer once the volume fraction of each phase is determined.

FIG. 5 is an example diagram showing a database of area fraction for each phase in the embodiment. FIG. 5 shows a part of the database in table form. The database contains measured results correlating the Al-Zn-Mg components with the area fraction SP (≈ volume fraction VP) of each phase. Using the database, the composition of the coating layer, and the molecular weight of each phase, the prediction unit 13 calculates the weight and molar quantity of each phase constituting the coating layer.

To create the database shown in FIG. 5, coating layers corresponding to the Zn-Al-Mg coating layer shown in FIG. 2 were prepared. Cross-sectional observations using EPMA (electron probe micro analyzer) were performed on the fabricated coating layers. The proportion of each phase was calculated such that it could be classified into values close to the composition values of each phase. Based on the calculated proportion of each phase, the area fraction SP (≈ volume fraction VP) of each phase was calculated.

A laboratory molten bath coating simulator was used to prepare the coated steel sheets for database creation. This molten bath coating simulator is capable of complete nitrogen replacement through testing. It enables execution of a series of coating operations under oxygen-free condition (less than 5 ppm) and allows monitoring of temperatures during the process.

For establishing the coating bath, a Zn-Al-Mg alloy was produced via vacuum melting using metal with a purity of 99% or higher. The base steel sheet for the coated steel sheet was a 1.2 mm cold-rolled steel sheet for general structural use (JIS G 3141). To suppress reactivity between iron (Fe) and the coating layer, the coating surface, which had been thoroughly degreased, cleaned, and pickled, was sequentially vapor-deposited with "Cr: 0.1 g/m²" and "Ni: 0.1 g/m²".

Using this as the base steel sheet to be coated, the base steel sheet to be coated was heated at 10°C/sec to 800°C. A 20% H₂-N₂ gas was then blown onto the base steel sheet to be coated and held for 60 seconds, performing surface reduction on the base steel sheet to be coated. After cooling the base steel sheet to be coated to the coating bath temperature, the base steel sheet to be coated was immersed in the coating bath for 1 second. After the coated steel sheet was withdrawn from the coating bath, the coated steel sheet was wiped with N₂ gas to control the coating layer thickness to 25 µm. The flow rate of the blown N₂ gas was controlled to achieve a time of 20 seconds from the melting point to 380°C (solidification completion temperature). The bath temperature was set to the melting point (= liquidus temperature as described in Reference Document 1) + 20°C.

In the vapor deposition process, the formation of an intermetallic compound layer containing Fe and Al between the coating bath and the coated steel sheet was suppressed. As a result, the coating layer's composition was nearly identical to that of the coating bath, eliminating the influence of Fe. However, because the coating layer is extremely thin, Fe diffusion into the coating layer is inhibited during immersion of the coated steel sheet in the coating bath, but Fe immediately diffuses back into the coating bath after the coating process. Consequently, these traces (Ni layer, Cr layer) scarcely remain in the coating layer, and the interfacial alloy layer is hardly present in the coating layer. Therefore, the influence of the base steel on the coating alloy layer is minimized.

The 20-second cooling refers to the typical time (20 seconds) from immersion in the coating bath until reaching the top roll. To prevent the coating bath from wrapping onto the top roll, so that the coated steel sheet passes after complete solidification, the temperature of the coated steel sheet is adjusted by gas blowing during the coating solidification process. Additionally, a 20 mm square coated steel sheet is cut out from the center of the coated steel sheet. The coated steel sheet is embedded in a resin in the vertical direction, and then the coated steel sheet is polished.

EPMA observation was performed on the polished cross-section of the coating layer of a coated steel sheet. Here, it was confirmed that the Al-Fe alloy layer and other components remained near the interface and that the coating bath composition corresponds to the coating layer composition; the thickness of the coating layer was also confirmed. Scanning electron microscope (SEM) images were obtained for the field of view where the coating layer thickness was confirmed, and then, for the positions where SEM images were obtained, EPMA was used to generate Zn, Al, and Mg compositional map images (EPMA maps). From these, the area fractions of each phase were determined using a database.

Typically, the coating layer solidifies from the surface, and consequently, the final solidification portion during the solidification process exists near the interface between the base steel and the coating layer. This results in the formation of a compositionally segregated structure in the horizontal cross-section of the coating layer. Therefore, the area fraction of each phase is measured in the cross-sectional direction of the coating layer.

To confirm the area fraction of each phase, the coating layer was examined at 500× magnification using a scanning electron microscope, and measurements of the area fraction for each phase were repeated until the total field of view of the coating layer reached 100,000 µm. Partial images of the coating layer were extracted from the EPMA image, and each extracted partial image was divided into a 5 µm × 5 µm grid.

A phase in which Al and Zn are detected, the content of Zn is 16.5 at% or less, and the content of other components is 5 at% or less is classified as the 1st phase "Pure_A1 phase".

A phase in which Al and Zn are detected and the Zn content is 16.5 to 37.75 at% is classified as the 2nd phase "Al-Zn(α) phase".

A phase in which Zn and Al are detected and the Zn content is 37.75 to 59 at% is classified as the 3rd phase "Zn-Al(β) phase".

A phase in which Zn and Al are detected and the Zn content is 59 to 98.4 at% is classified as the 3rd phase "Zn-Al(β) phase" and the 4th phase "Pure_Zn(η) phase".

A phase in which the Mg content is 33.3 at% (±3 at%) and the Zn content is 66.6 at% (±3%) is classified as the 5th phase "MgZn2 phase" (single phase).

A phase in which Zn, Al, and Mg are detected, the Zn content is 80 at% or more, the Mg content is 1 to 10 at%, and the Al content is 5 at% to less than 15 at% is classified as the 6th phase "ternary eutectic structure".

As shown above, phases can often be classified based on their Zn content. Exceptionally, the interface between the coating layer and the steel, as well as the resin interface, may be classified as "others". Therefore, the area fraction of each phase is confirmed within the range not classified as others.

Below, the subscript of a character denotes the phase number (n-th phase). For example, the area fraction "SP₁" represents the area fraction of the 1st phase. For example, the volume fraction "VP₂" represents the volume fraction of the 2nd phase.

The area fractions of SP₁ to SP₆ (≈ volume fraction VP₁ to VP₆) can be confirmed in the database. Therefore, based on the total volume "V = V₁ + V₂ + V₃ + V₄ + V₅ + V₆" of each phase constituting the coating layer and the volume fraction "VPₙ" corresponding to the density "ρₙ" of the n-th phase, the prediction unit 13 calculates the volume "Vₙ" of the n-th phase as shown in Equation (3).${V}_{n} = {VP}_{n} \div 100 \div V$

Based on the density "ρₙ" of the n-th phase and the volume "Vₙ" of the n-th phase, the prediction unit 13 calculates the content (weight) "mₙ" of each phase in the coating layer, as shown in Equation (4).${m}_{n} = {V}_{n} \times {ρ}_{n}$

Furthermore, the total content (by weight) "M" of each phase in the coating layer is expressed as in Equation (5).$M = {m}_{1} + {m}_{2} + {m}_{3} + {m}_{4} + {m}_{5} + {m}_{6}$

When indicating as "mass%", the prediction unit 13 converts the content (weight) "mₙ" of each phase to the content (weight) "MSₙ" (mass% indication) of each phase using Equation (6).${MS}_{n} = {m}_{n} / M \times 100$

The prediction unit 13 calculates the weight "RMₙ" of the n-th phase contained in the coating layer by multiplying the content (weight) "MSₙ" of each phase by the total coating weight "ρL" of the coating layer, as shown in Equation (7).${RM}_{n} = ρL \times {MS}_{n}$

Furthermore, the prediction unit 13 may calculate the molar quantity "molₙ" of the n-th phase based on the weight "RMₙ" and molecular weight "Nₙ" of the n-th phase, as shown in Equation (8).${mol}_{n} = {RM}_{n} / {N}_{n}$

The total molar quantity "MOL" is expressed as shown in Equation (9).$MOL = {mol}_{1} + {mol}_{2} + {mol}_{3} + {mol}_{4} + {mol}_{5} + {mol}_{6}$

The prediction unit 13 may calculate the molar percentage "mol%ₙ" of the n-th phase using Equation (10).${mol %}_{n} = {mol}_{n} / MOL$

In this manner, the prediction unit 13 calculates the proportion of each phase in the coating layer. Based on the proportion of each phase, the prediction unit 13 uniformly distributes each phase within the corrosion morphology model of the coating layer. The prediction unit 13 calculates the corrosion weight loss for each of Stage I "τ1" and Stage II "τ2". This enables the determination of the corrosion resistance (service life) of the coating layer. Note that there exists a specific sequence in which each phase corrodes.

In exposure tests and corrosion acceleration tests (such as composite cycle corrosion tests), each phase corroded in order of increasing corrosion potential. Specifically, the applicant confirmed, by cross-sectional observations of the coating layer in numerous corrosion tests, that corrosion proceeded in the following order (corrosion order): the 3rd phase "Zn-Al(β) phase", the 6th phase "ternary eutectic structure", the 5th phase "MgZn2 phase", the 4th phase "Pure_Zn(η) phase", the 2nd phase "Al-Zn(α) phase", and the 1st phase "Pure_Al phase".

Next, an example of the operation of the prediction device 1 will be described.

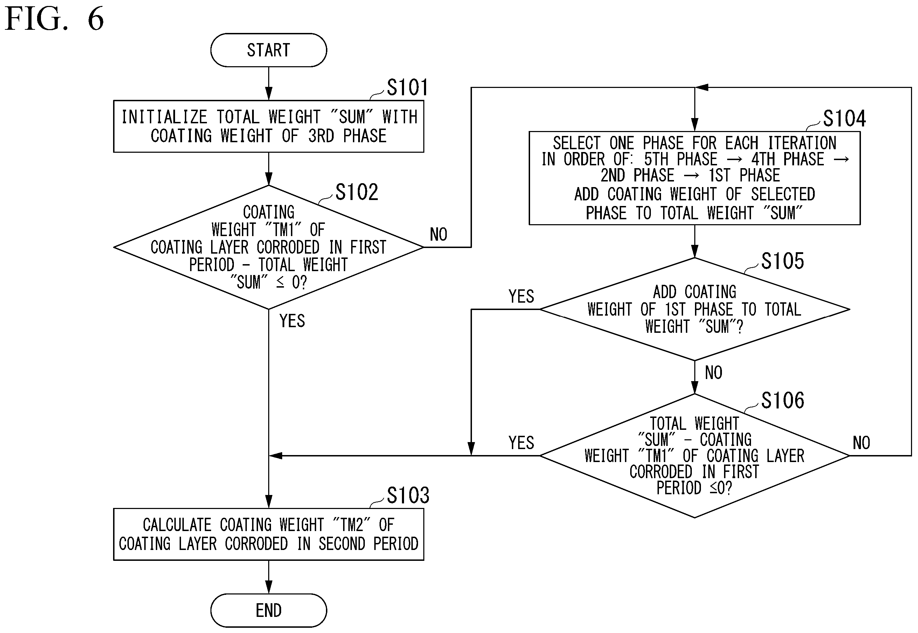

FIG. 6 is a flowchart showing an example of the process for calculating the coating weight of each phase in the embodiment. Since the phases corrode in the order described above, the proportion of the phase with the lower corrosion potential increases during Stage I "τ1". During Stage II "τ2", the proportion of the phase with the higher corrosion potential increases. The prediction unit 13 calculates the proportion of the coating weight "TM1" of the coating layer that corrodes during Stage I "τ1" for each phase and the proportion of the coating weight "TM2" of the coating layer that corrodes during Stage II "τ2" for each phase, as follows.

The variable "SUM" described below represents the total weight. The total weight "SUM" is substituted with the total weight in accordance with the processing performed at each step. The total weight "SUM" is used to determine whether the end timing of Stage I "τ1" has been reached. Specifically, the total weight "SUM" is used to determine whether corrosion has reached the boundary between the portion of the coating layer corroded during Stage I "τ1" and the portion of the coating layer corroded during Stage II "τ2". Here, the end timing of Stage I "τ1" is determined based on the proportion "G" of the coating layer corroded during Stage I "τ1".

The prediction unit 13 initializes the total weight "SUM" (variable) with the coating weight of the 3rd phase (Step S101). The prediction unit 13 determines whether the coating weight "TM1" of the coating layer corroding during the first period minus the total weight "SUM" is 0 or less (Step S102).

If the coating weight "TM1" of the coating layer corroded during the first period minus the total weight "SUM" is 0 or less (Step S102: YES), the prediction unit 13 calculates the coating weight "TM2" of the coating layer corroded during the second period. Here, the coating weight "TM2" is given by "TM2 = SUM - TM1 + RM₆ + RM₅ + RM₄ + RM₂ + RM₁" (Step S103-1).

If the coating weight "TM1" of the coating layer corroded during the first period minus the total weight "SUM" is greater than 0 (Step S102: NO), the prediction unit 13 selects one phase in the order of the 5th phase → the 4th phase → the 2nd phase → the 1st phase for each iteration starting from Step S106. The prediction unit 13 adds the coating weight "RMₙ" of the selected n-th phase to the total weight "SUM" (Step S104). The prediction unit 13 determines whether the coating weight "RM₁" of the 1st phase has been added to the total weight "SUM" (Step S105).

When it is determined that the total weight "SUM" includes the coating weight of phases other than the 1st phase (Step S105: YES), the prediction unit 13 determines whether the total weight "SUM" minus the coating weight "TM1" of the coating layer corroded during the first period is 0 or less (Step S106). If it is determined that the total weight "SUM" minus the coating weight "TM1" of the coating layer corroded during the first period is greater than 0 (Step S106: NO), the prediction unit 13 returns the processing to Step S104.

If the total weight "SUM" minus the coating weight "TM1" of the coating layer corroded during the first period is determined to be 0 or less (Step S106: YES), the prediction unit 13 returns the processing to Step S103. Here, if the coating weight "RM₆" of the 6th phase was added to the total weight "SUM" in Step S104, the coating weight "TM2" is given by "TM2 = SUM - TM1 + RM₅ + RM₄ + RM₂ + RM₁" (Step S103-2). If the coating weight "RM₅" of the 5th phase was added to the total weight "SUM" in Step S104, the coating weight "TM2" is given by "TM2 = SUM - TM1 + RM₄ + RM₂ + RM₁" (Step S103-3). If the coating weight "RM₄" of the 4th phase was added to the total weight "SUM" in Step S104, the coating weight "TM2" is given by "TM2 = SUM - TM1 + RM₂ + RM₁" (Step S103-4). If the coating weight "RM₂" of the 2nd phase was added to the total weight "SUM" in Step S104, the coating weight "TM2" is given by "TM2 = SUM - TM1 + RM₁" (Step S103-5).

If it is determined that the coating weight "RM" of the 1st phase has been added to the total weight "SUM" (Step S105: YES), the prediction unit 13 returns the processing to Step S103. Here, the coating weight "TM2" is given by "TM2 = SUM - TM1" (Step S103-6).

### (Corrosion Rate)

The prediction unit 13 calculates the service life of the coating layer based on the coating weight "TM1" and corrosion rate "r1" in Stage I "τ1", and on the coating weight "TM2" and corrosion rate "r2" in Stage II "t2". Hereafter, the corrosion rate of the n-th phase in Stage I "τ1" is denoted as "r1ₙ". The corrosion rate of the n-th phase in Stage II "τ2" is denoted as "r2ₙ".

In corrosion acceleration tests (composite cycle corrosion tests), the corrosion rate is the corrosion weight loss per specified cycle. Therefore, the units for the corrosion rates "r1" and "r2" of the coating layer are "g/m²/cycle". In exposure tests, etc., the units for the corrosion rates "r1" and "r2" of the coating layer are "g/m²/year (day, month)", etc. The corrosion rates "r1" and "r2" of the coating layer vary depending on the corrosion environment of the corrosion acceleration test or exposure test.

Single-phase metal test specimens were prepared by rapidly quenching the alloy compositions of each phase to suppress the formation of compositional bands. The corrosion rates of each phase were determined by subjecting these prepared metal test specimens to various corrosion tests.

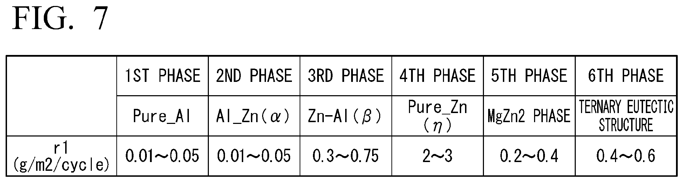

FIG. 7 shows an example of the corrosion rates for each phase during the first period in the embodiment. In the composite cycle corrosion tests (JIS H 8502 JASO M609-91), the corrosion rates are as shown in FIG. 7. Regarding the corrosion rate "r2", the corrosion acceleration depends on the composition of the steel sheet. When general mild steel is used in the test, the corrosion rate "r2" in the composite cycle corrosion tests (JIS H 8502 JASO M609-91) is often 30 to 50 times the corrosion rate "r1".

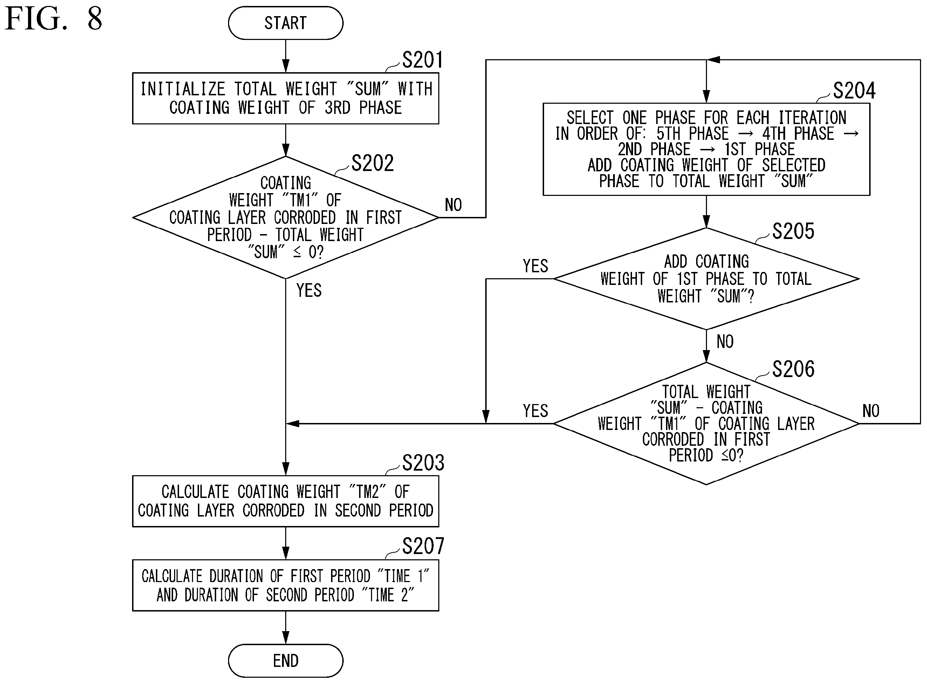

FIG. 8 is a flowchart illustrating an example of a process for calculating the duration "TIME1" of the period (first period) during which partial corrosion can be confirmed on the surface of the coating layer, and the duration "TIME2" of the period (second period) after partial corrosion has reached the interface between the coating layer and the steel sheet, in the embodiment. Each step from Step S201 to Step S206 shown in FIG. 8 is the same as each step from Step S101 to Step S106 shown in FIG. 6.

If the coating weight "TM1" of the coating layer corroded during the first period minus the total weight "SUM" is 0 or less (Step S202: YES), the prediction unit 13 calculates the coating weight "TM2" of the coating layer corroded during the second period (Step S203-1). The prediction unit 13 also calculates the duration "TIME1" of the first period and the duration "TIME2" of the second period. Here, "TIME = SUM/r1₃". Furthermore, "TIME2 = (SUM - TM1) / r2₃ + RM₆ / r2₆ + RM₅ / r2₅ + RM₄ / r2₄ + RM₂ / r2₂ + RM₁ / r2₁" (Step S207-1).

If the total weight "SUM" minus the coating weight "TM1" of the coating layer corroded during the first period is determined to be 0 or less (Step S206: YES), the prediction unit 13 returns the processing to Step S203. Furthermore, the prediction unit 13 calculates the duration "TIME1" for the first period and the duration "TIME2" for the second period. Here, if the coating weight "RM₆" of the 6th phase was added to the total weight "SUM" in Step S204, "TIME1 = RM₃/r1₃ + (SUM - RM₃)/r1₆". Furthermore, "TIME2 = (SUM - TM1)/r2₆ + RM₅/r2₅ + RM₄/r2₄ + RM₂/r2₂ + RM₁/r2₁" (Step S207-2). If the total weight "SUM" has been increased by the coating weight "RM₅" of the 5th phase in Step S204, "TIME1 = RM₃/r1₃ + RM₆/r1₆ + (SUM - RM₃ - RM₆)/r1₅". Furthermore, "TIME2 = (SUM - TM1)/r2₅ + RM₄/r2₄ + RM₂/r2₂ + RM₁/r2₁" (Step S207-3). If the 4th phase coating weight "RM₄" is added to the total weight "SUM" in Step S204, then "TIME = RM₃/r1₃ + RM₆/r1₆ + RM₅/r1₅ + (SUM - RM₃ - RM₆ - RM₅)/r1₄". Furthermore, "TIME2 = (SUM - TM1)/r2₄ + RM₂/r2₂ + RM₁/r2₁" (Step S207-4). If the 2nd phase coating weight "RM₂" has been added to the total weight "SUM" in Step S204, then "TIME1 = RM₃/r1₃ + RM₆/r1₆ + RM₅/r1₅ + RM₄/r1₄ + (SUM - RM₃ - RM₆ - RM₅ - RM₄)/r1₂". Furthermore, "TIME2 = (SUM - TM1)/r2₂ + RM₁/r2₁" (Step S207-5).

If it is determined that the total weight "SUM" includes the coating weight "RM₁" of the 1st phase (Step S205: YES), the prediction unit 13 returns the processing to Step S203. The prediction unit 13 also calculates the duration "TIME1 " of the first period and the duration "TIME2" of the second period. Here, "TIME1 = RM₃/r1₃ + RM₆/r1₆ + RM₅/r1₅ + RM₄/r1₄ + RM₂/r1₂ + (SUM - RM₃ - RM₆ - RM₅ - RM₄ - RM₃)/r1₁". Furthermore, "TIME2 = (SUM - TM1)/r2₁" (Step S207-6).

In this manner, the prediction unit 13 calculates the period "TIME1" for Stage I "τ1" and "TIME2" for Stage II "τ2". The prediction unit 13 calculates the corrosion resistance (service life) of the coating layer as "TIME = TIME1 + TIME2".

As described above, the prediction unit 13 (proportion calculation unit) calculates, based on respective contents of multiple components contained in a coating layer, a proportion (first proportion) during a first period for each phase constituting the coating layer, and a proportion (second proportion) during a second period for each phase constituting the coating layer. The prediction unit 13 (first period calculation unit) calculates, based on a corrosion order of the each phase, the first proportion of the each phase, and a first corrosion rate of the each phase, a duration of the first period until a part of corrosion of the coating layer reaches a base steel. The prediction unit 13 (second period calculation unit) calculates, based on a corrosion order of the each phase, the second proportion of the each phase, and a second corrosion rate of the each phase, a duration of the second period following the first period. The second corrosion rate is a rate in which the first corrosion rate is corrected in accordance with a corrosion acceleration of the coating layer due to a corrosion prevention of the base steel. The prediction unit 13 (prediction result calculation unit) calculates a sum of the duration of the first period and the duration of the second period as a prediction result of corrosion resistance of the coating layer.

In this manner, the prediction unit 13 calculates the duration of the corrosion period (service life) based on the corrosion order of each phase, the proportion of each phase, and the corrosion rate of each phase. This enables improved accuracy in predicting the corrosion resistance of the coating layer. End users of coated steel sheets can efficiently select materials based on the prediction results. Furthermore, the time required for approval of coated steel sheet adoption can be shortened, enabling savings in labor costs.

In this manner, the prediction unit 13 may separate the corrosion period (service life) into Stage I and Stage II. That is, the prediction unit 13 may calculate the corrosion period in Stage I and the corrosion period in Stage II separately. This enables further improvement in the accuracy of predicting the corrosion resistance of the coating layer.

### (Modification example)

To enhance the accuracy of predicting the corrosion resistance (service life) of the coating layer, actual corrosion test data may be incorporated into the prediction results. By adjusting the predicted service life "TIME" to match the service life "TIME'" (actual data) obtained from corrosion tests using a correction function "f" (TIME' = f(TIME)), high prediction accuracy is achieved.

Examples of data points obtained in the corrosion test are shown below.
- (Zn, Al, Mg) = (80, 13, 7) at%, coating thickness 20 µm, red rust generation cycle = 210
- (Zn, Al, Mg) = (80, 17, 8) at%, coating thickness 20 µm, red rust generation cycle = 240
- (Zn, Al, Mg) = (70, 22, 8) at%, coating thickness 20 µm, red rust generation cycle = 300
- (Zn, Al, Mg) = (70, 24, 10) at%, coating thickness 20 µm, red rust generation cycle = 300
- (Zn, Al, Mg) = (52, 34, 13) at%, coating thickness 20 µm, red rust generation cycle = 840
- (Zn, Al, Mg) = (25, 73, 2) at%, coating thickness 20 µm, red rust generation cycle = 840

The prediction unit 13 may correct the predicted service life results by using machine learning techniques (e.g., supervised learning), based on an increased number of data points obtained through corrosion tests.

Although the embodiments of the present invention have been described in detail with reference to the accompanying drawings, the specific configurations are not limited to the embodiments, and designs and the like within the scope of the present invention are also included.

### REFERENCE SIGNS LIST

1 Prediction device, 11 Storage device, 12 Storage unit, 13 Prediction unit, 14 Display unit, 101 Coating layer, 102 Steel, 103 Coating layer

## Claims

1. A prediction device comprising a prediction unit, wherein the prediction unit is configured to calculate, based on respective contents of multiple components contained in a coating layer, a first proportion during a first period and a second proportion during a second period, for each phase constituting the coating layer, calculate, based on a corrosion order of the each phase, the first proportion of the each phase, and a first corrosion rate of the each phase, a duration of the first period until a part of corrosion of the coating layer reaches a base steel, calculate, based on a corrosion order of the each phase, the second proportion of the each phase, and a second corrosion rate of the each phase, a duration of the second period following the first period, and calculate a sum of the duration of the first period and the duration of the second period as a prediction result of corrosion resistance of the coating layer.

2. The prediction device according to Claim 1, wherein the second corrosion rate is a rate in which the first corrosion rate is corrected in accordance with a corrosion acceleration of the coating layer due to a corrosion prevention of the base steel.

3. A prediction method executed by a prediction device, the method comprising steps of calculating, based on respective contents of multiple components contained in a coating layer, a first proportion during a first period and a second proportion during a second period, for each phase constituting the coating layer, calculating, based on a corrosion order of the each phase, the first proportion of the each phase, and a first corrosion rate of the each phase, a duration of the first period until a part of corrosion of the coating layer reaches a base steel, calculating, based on a corrosion order of the each phase, the second proportion of the each phase, and a second corrosion rate of the each phase, a duration of the second period following the first period, and calculating a sum of the duration of the first period and the duration of the second period as a prediction result of corrosion resistance of the coating layer.

4. A prediction program for causing a computer to execute processes of calculating, based on respective contents of multiple components contained in a coating layer, a first proportion during a first period and a second proportion during a second period, for each phase constituting the coating layer, calculating, based on a corrosion order of the each phase, the first proportion of the each phase, and a first corrosion rate of the each phase, a duration of the first period until a part of corrosion of the coating layer reaches a base steel, calculating, based on a corrosion order of the each phase, the second proportion of the each phase, and a second corrosion rate of the each phase, a duration of the second period following the first period, and calculating a sum of the duration of the first period and the duration of the second period as a prediction result of corrosion resistance of the coating layer.
